# FASCICULE DE BREVET EUROPEEN

(11) **EP 1 066 011 B1**
(45) Date de publication et mention de la délivrance du brevet: **13.05.2009**
(21) Numéro de dépôt: 99936026.6
(22) Date de dépôt: 26.02.1999
(51) Int. Cl.: A61G 15/10, A61B 19/10

(54) **DISPOSITIF POUR INTERVENTION CHIRURGICALE DESTINE A ASSURER UNE PROTECTION DU PATIENT CONTRE LES CONTAMINATIONS, NOTAMMENT EN CHIRURGIE DENTAIRE**
CHIRURGISCHES GERÄT ZUM SCHUTZ DES PATIENTEN GEGEN KONTAMINATION, INSBESONDERE BEI ZAHNCHIRURGISCHEN EINGRIFFEN
SURGICAL PROCEDURE DEVICE FOR PROTECTING THE PATIENT AGAINST CONTAMINATION, IN PARTICULAR IN DENTAL SURGERY

(30) Priorité: 26.02.1998 FR 9802331
(43) Date de publication de la demande: 10.01.2001
(73) Titulaire: Ravineau, Michel, 41700 Cour Cheverny (FR)
(72) Inventeur: Ravineau, Michel, 41700 Cour Cheverny (FR)
(74) Mandataire: Remy, Vincent Noel Paul
(86) Numéro de dépôt international: PCT/FR1999/000441
(87) Numéro de publication internationale: WO 1999/043281

(56) Documents cités:
- US-A- 3 137 387
- US-A- 4 334 529
- US-A- 4 553 538
- US-A- 5 464 025

## Description

La présente invention concerne un dispositif pour intervention chirurgicale destiné à assurer une protection du patient contre les contaminations, notamment en chirurgie dentaire. Un tel dispositif est décrit dans US-A-3 137 387.

La protection contre les contaminations est une préoccupation croissante, notamment dans le milieu médical.

Pour les cabinets dentaires, on a imaginé un certain nombre de solutions de protection des matériels, consistant notamment à recouvrir ces derniers de films étanches jetables que le praticien est censé remplacer entre deux patients.

Ces protections présentent deux inconvénients majeurs.

D'une part, leur efficacité n'est pas certaine car si le film par lui-même est parfaitement étanche, il doit néanmoins laisser des passages pour le raccordement des appareils à différentes sources d'énergie ou de fluides.

En outre, la manipulation des appareils par le praticien oblige ce dernier, ne serait-ce que lors du remplacement des films protecteurs, à toucher simultanément les films protecteurs souillés et les appareils à protéger.

D'autre part, la mise en place de tels films protecteurs requiert de très nombreuses manipulations non sans risque d'erreur d'asepsie, manipulations qui sont très longues à exécuter et qui obligent le praticien à prévoir un intervalle de temps important entre deux patients.

La présente invention vise à résoudre notamment ces inconvénients et bien d'autres qui apparaîtront à la lecture de la description.

La présente invention a pour objet un dispositif pour interventions chirurgicales destiné à assurer une protection des patients contre les contaminations, notamment en chirurgie dentaire, caractérisé par le fait qu'il comporte les caractéristiques de la revendication 1.

En d'autres termes, l'invention réalise une barrière entre le patient, qui est potentiellement contaminé, et les différents appareils propres ou stériles installés de façon inamovible dans la salle d'opération.

En effet, dans le cas de la chirurgie dentaire par exemple, où l'intervention a lieu dans la bouche du patient, le chirurgien utilise des instruments stérilisables ou jetables qui demeurent du côté de la face supérieure de la feuille protectrice, c'est-à-dire dans le milieu contaminé, en étant éventuellement raccordés au tuyau flexible qui traverse la feuille protectrice de manière étanche tandis que le siège, le support et tout autre dispositif installé de façon inamovible dans le cabinet, sont situés dans une zone propre ou stérile et sont protégés du milieu contaminé par la feuille protectrice.

Une fois l'intervention achevée, le praticien se débarrasse des instruments jetables qu'il a utilisés, stérilise ceux qui peuvent l'être, et remplace la feuille protectrice solidaire du tuyau flexible souillé par une feuille protectrice et un tuyau flexible stériles.

Dans un mode de réalisation particulier de l'invention, la feuille protectrice recouvre également un siège sur lequel le patient prend place pour subir l'intervention.

On entend par siège tout support sur lequel un patient peut prendre place pour subir une intervention. Il peut s'agir en particulier d'une table d'opération.

Ainsi, le dispositif selon l'invention peut être utilisé dans un bloc opératoire pour toute intervention chirurgicale, en protégeant la table sur laquelle se trouve le patient et tous les appareillages et robinets installés dans le bloc.

Le raccordement du tuyau flexible à une source de fluide s'effectue de préférence par un raccord rapide et sans contact direct avec cette source, que celle-ci soit un robinet, un conduit ou un récipient.

Par exemple, la source peut fournir un gaz anesthésiant, un produit chimique pour intraveineuse ou pour un cathéter.

Le tuyau flexible peut également être raccordé à une source de vide, pour réaliser une aspiration de fluides, notamment de fluides corporels.

La feuille protectrice et ses tuyaux flexibles sont avantageusement prévus pour être facilement pliés, dépliés et repliés, de manière à pouvoir être transportés stockés avant utilisation, utilisés et à nouveau stockés en tant que déchets après utilisation.

Avant utilisation, on stérilise de préférence le dispositif selon l'invention pour le stocker plié sous vide ou dans une atmosphère inerte sous plastique.

Dans un mode de réalisation particulier de l'invention, le dispositif comporte, formée dans la feuille protectrice, au moins une partie en creux pénétrant, lorsque la feuille protectrice est mise en place sur le support, dans un réceptacle du support apte à recevoir des instruments opératoires.

On comprend que la partie en creux de la feuille protectrice prend place dans le réceptacle et protège ce dernier de toute contamination, les différents instruments utilisés par le chirurgien lors de l'intervention étant stockés dans le réceptacle au cours de l'intervention.

Une fois l'intervention terminée, la partie en creux qui protège le réceptacle est éliminée avec la feuille protectrice.

Le réceptacle protégé par la partie en creux de la feuille protectrice constitue un véritable support qui remplace le plateau sur lequel le praticien fait habituellement préparer ses instruments stérilisés, aux différences près que dans le réceptacle selon l'invention, les instruments sont stockés en position verticale, ce qui évite tout risque de chute et de blessure accidentelle du praticien ; que tous les instruments, c'est-à-dire ceux qui sont fixés aux tuyaux flexibles ainsi que les instruments à main tels que miroir, sonde, davier ou précelle sont tous réunis au même endroit, ce qui facilite l'organisation du praticien ; que le stockage des instruments occupe une place restreinte dans le champ de travail du praticien ; et que les instruments ne sont pas accessibles à la vue du patient, ce qui contribue à le rassurer.

Dans une variante particulière, le dispositif selon l'invention comprend plusieurs réceptacles sur le support et un nombre correspondant de parties en creux sur la feuille protectrice, ce qui permet de ranger les instruments par catégorie ou de réserver un réceptacle au stockage de déchets.

Le dispositif selon l'invention assure donc une protection efficace du matériel non stérilisable de la salle d'intervention moyennant une manipulation très simple et très rapide.

Dans un mode de réalisation particulier, la feuille protectrice comporte un opercule détachable au-dessus de sa partie en creux, constituant un témoin d'inviolabilité dont la fonction est de garantir le patient et le praticien que le dispositif installé sur le siège pour les soins n'a encore jamais été utilisé. Cet opercule est de préférence coloré de manière visible.

Un mode de gestion de tels dispositifs pourrait consister en ce que le praticien remette à son patient l'opercule du dispositif, revêtu de son cachet et/ou de la signature, le patient pouvant utiliser cet opercule comme justificatif des soins qu'il a reçus pour en obtenir le remboursement auprès d'un organisme d'assurance.

Dans le même ordre d'idée, le dispositif complet plié éventuellement stérilisé et emballé sous plastique pourrait être acheté par le patient en pharmacie sur ordonnance et fourni au praticien par le patient au moment de l'intervention. Ainsi, le praticien n'aurait pas à gérer lui-même ses stocks de feuilles protectrices propres, éventuellement stérilisées.

Dans un mode de réalisation préféré de l'invention, la feuille protectrice et le tuyau flexible constituent un ensemble d'un seul tenant réalisé en un matériau peu coûteux de sorte que cet ensemble peut être jeté après chaque utilisation.

Dans une variante préférée, le dispositif selon l'invention comporte deux tuyaux flexibles sur la feuille protectrice, à savoir un tuyau flexible pour une alimentation en eau, un tuyau flexible pour une alimentation en air comprimé, destinés à se raccorder à des conduits d'alimentation (12, 13) sur le support.

De préférence, le dispositif comporte, à la base des deux tuyaux flexibles, un raidisseur qui réunit lesdites bases et les solidarise entre elles.

Dans une autre variante, compatible avec la précédente, le dispositif comporte un tuyau flexible d'aspiration sur la feuille protectrice destiné à se raccorder à un conduit d'aspiration sur le support.

Dans une version avantageuse réunissant des tuyaux flexibles d'alimentation en eau et en air et un tuyau flexible d'aspiration selon les deux variantes ci-dessus, on prévoit de séparer les embouchures des tuyaux flexibles d'alimentation de celle du tuyau flexible d'aspiration sur la feuille protectrice par une distance importante ou par une cloison de séparation de manière à éviter que des gouttelettes de liquide contaminé provenant du tuyau flexible d'aspiration ne viennent souiller les embouchures des conduits d'alimentation au moment du retrait de la feuille protectrice.

Dans une autre variante, compatible avec les précédentes, le dispositif comporte une ligne électrique constituée par un ou plusieurs fils conducteurs sur la feuille protectrice, traversant cette dernière de façon étanche comme les tuyaux flexibles et munie de broches de contact aptes à coopérer avec une prise électrique du support lorsque la feuille protectrice est mise en place.

Une telle variante est avantageuse car elle permet d'utiliser tous les appareillages électriques modernes tels que micromoteurs, lampes à photopolymériser, bistouris électriques, fraises à ultrasons, éclairages de dents en bout de turbines, avec le dispositif selon l'invention dans de bonnes conditions de sécurité vis-à-vis des problèmes de contamination entre patients ou entre le patient et le praticien. Cet avantage apparaît d'autant plus précieux en chirurgie implantaire, en parodontologie, en stomatologie, domaines dans lesquels les interventions occasionnent des saignements importants.

Dans une autre variante, également compatible avec les précédentes, le dispositif comporte au moins une gaine de protection constituée par un tuyau flexible de très gros diamètre raccordé de façon étanche par une de ses extrémités à la face de la feuille protectrice opposée au support et débouchant sur l'autre face de ladite feuille, pour permettre au praticien de manipuler des appareils non stérilisables, en les engageant dans la gaine.

La gaine peut être obturée à son extrémité pour constituer un étui qui protège complètement l'appareil qui y est introduit.

Le support est de préférence relié à au moins une pédale de commande de la circulation du ou des fluides dans les conduits dudit support et donc dans les tuyaux flexibles.

Avantageusement, la partie en creux de la feuille protectrice comporte un fond métallisé, tandis que le réceptacle du support comporte un fond aimanté.

On comprend que l'aimant situé au fond du réceptacle attire le fond magnétisé de la partie en creux de la feuille, facilite sa mise en place et assure son maintien en position, ce qui contribue au maintien de la feuille sur le support.

Pour faciliter le pliage de la feuille, la partie en creux est avantageusement conformée en accordéon de manière à pouvoir s'allonger facilement et se rétracter tout aussi aisément.

Dans le cas d'un cabinet dentaire, le support est fixé de préférence à la têtière du fauteuil du patient, qui constitue le siège au sens de l'invention.

Selon l'invention, il est avantageux qu'au moins un tuyau flexible, et idéalement tous, comporte un clapet anti-retour de manière à éviter toute contamination des conduits de circulation de fluide aboutissant au support par un phénomène d'aspiration inattendu.

Dans un mode de réalisation préféré, le tuyau flexible d'alimentation en eau se raccorde à un embout fixe monté à l'extrémité du conduit d'alimentation en eau et le tuyau flexible d'alimentation en air comprimé se raccorde à un embout mobile monté à l'extrémité du conduit d'alimentation en air comprimé et supporté par un bras pivotant autour de l'embout fixe, le support étant muni d'une lumière en regard de laquelle l'embout mobile se déplace lors du pivotement du bras, ladite lumière comportant une partie large permettant l'introduction du tuyau flexible d'alimentation en air comprimé et une partie étroite dont les bords pincent ledit tuyau flexible.

Dans une variante particulière de ce mode de réalisation, dans laquelle le dispositif est muni d'une ligne électrique, la prise électrique du support est solidaire du bras pivotant et le support comporte une seconde lumière qui permet à ladite prise de pivoter avec l'embout mobile tout en restant accessible depuis le dessus du support pour le branchement des broches de contact.

Avec ou sans ligne électrique, il est préférable, pour éviter toute intervention directe dans la zone stérile, que le bras pivotant soit équipé d'un moyen de rappel, par exemple un ressort, qui assure son retour en position de branchement, c'est-à-dire avec son embout mobile en regard de la partie large de la lumière pour l'introduction du tuyau flexible d'air comprimé. Une butée garantit alors le bon positionnement du bras pivotant rappelé en position de branchement.

Dans un mode de réalisation particulier de l'invention, le dispositif comporte, à l'extrémité du tuyau flexible, une prise universelle destinée à recevoir tout instrument nécessitant un fluide pour son fonctionnement, le tuyau flexible se raccordant à la prise universelle par simple emmanchement d'un tronçon d'extrémité du tuyau flexible dans un tube de la prise, et éventuellement pincement de la paroi extérieure dudit tuyau flexible.

Dans une variante particulière, la prise universelle est en outre raccordée aux fils conducteurs et comporte des moyens de branchement conventionnels pour le montage d'un instrument nécessitant notamment une alimentation électrique.

La prise universelle selon l'invention a donc pour fonction d'alimenter l'instrumentation rotative et de servir de support aux tuyaux d'air et d'eau pour les fonctions de séchage et de rinçage en bouche. Le fait que le ou les tuyaux flexibles se raccordent par simple emmanchement dans un tube rend en effet le démontage d'un tuyau pour sécher ou pour rincer en bouche, facile à réaliser.

Dans un autre mode de réalisation de l'invention, le dispositif comporte un embout dentaire constitué par un cylindre muni de deux orifices latéraux, de préférence d'axe oblique par rapport à l'axe du cylindre, l'une des extrémités du cylindre étant destinée à recevoir l'extrémité d'un tuyau flexible d'aspiration, l'un des deux orifices latéraux étant destiné à recevoir l'extrémité d'un tuyau flexible d'alimentation en eau, et l'autre orifice latéral étant destiné à recevoir l'extrémité d'un tuyau flexible d'alimentation en air comprimé.

De préférence, chaque tuyau flexible est apte à coulisser dans l'orifice latéral dans lequel il est engagé, de manière à pouvoir s'enfoncer dans l'embout pour faire saillie à l'extrémité de l'embout opposée à celle recevant le tuyau d'aspiration.

L'embout sert ainsi de moyen de préhension des tuyaux flexibles.

Dans une variante, l'embout comporte au moins un tube coudé flexible emmanché dans l'embout à l'opposé du tuyau flexible d'aspiration, ce tube coudé étant muni d'une crépine à son extrémité libre et étant destiné à être mis en place dans la bouche d'un patient pour aspirer sa salive.

L'embout ainsi équipé remplit la fonction de support des tuyaux flexibles du fait qu'il les réunit tous les trois à proximité de la bouche du patient, ce qui permet au praticien de saisir l'un desdits tuyaux flexibles et de le séparer de l'embout s'il désire l'utiliser seul, sans avoir à aller chercher le tuyau flexible à distance de la bouche du patient.

Le tube coudé étant emmanché à l'opposé du tuyau flexible d'aspiration, l'embout se trouve suspendu à la bouche du patient sensiblement en position verticale, ce qui est à la fois peu encombrant et ergonomique pour le praticien.

L'invention a également pour objet un embout tel que décrit ci-dessus.

Dans le but de mieux faire comprendre l'invention, on va en décrire maintenant des modes de réalisation donnés à titre d'exemples non limitatifs en référence au dessin annexé dans lequel :
- la figure 1 représente en perspective, une feuille protectrice selon un mode de réalisation de l'invention, vue du côté de sa face supérieure,
- la figure 2 est une vue en élévation du dispositif selon l'invention, lors de sa mise en place sur un fauteuil de cabinet dentaire,
- la figure 3 est une vue analogue à la figure 2 après mise en place de la feuille protectrice,
- la figure 4 est une vue partielle de dessus de la figure 3,

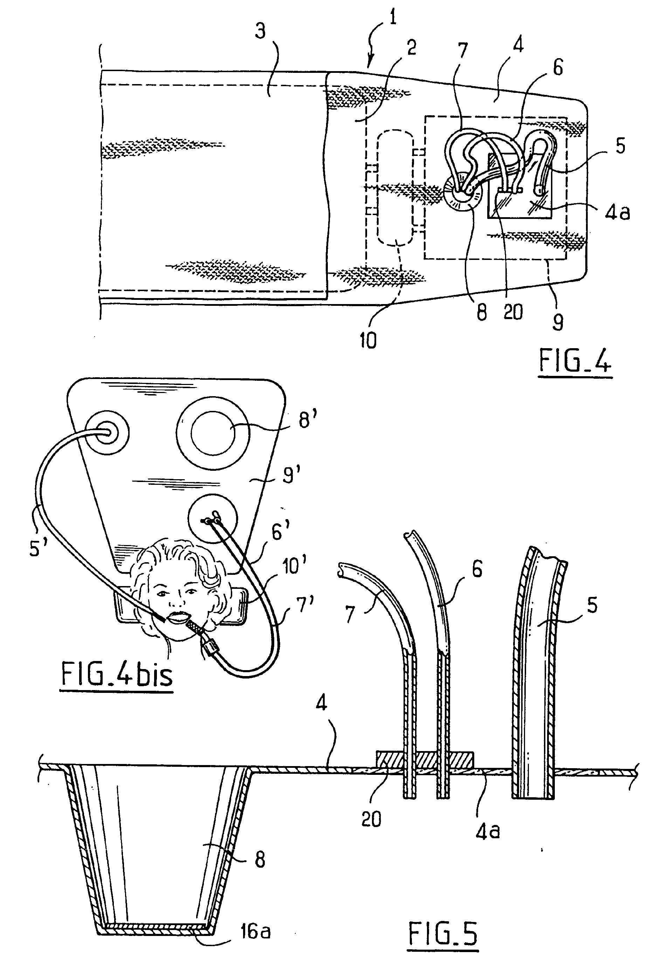

- la figure 4bis est analogue à la figure 4 mais représente une variante de répartition des éléments sur le support,

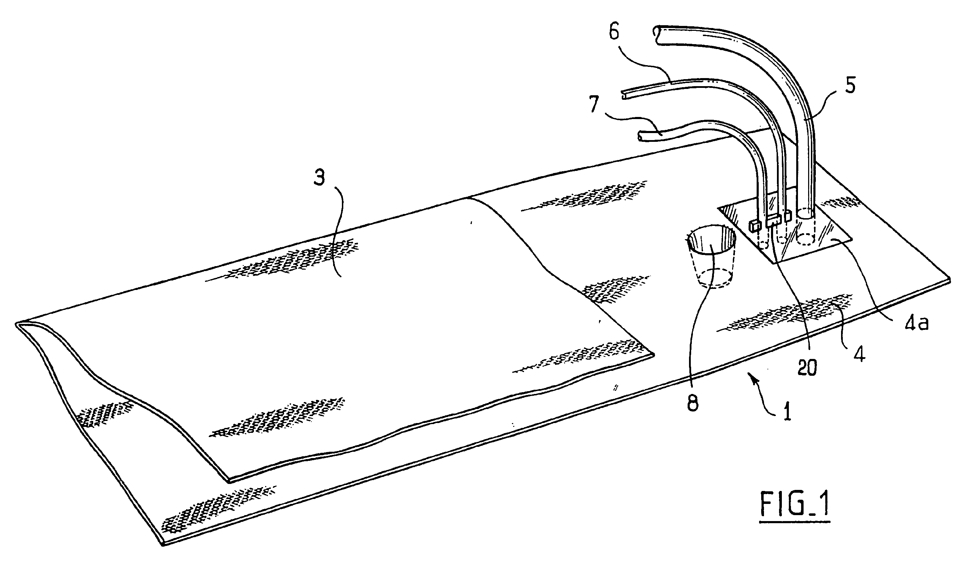

- la figure 5 est une vue de détail en coupe selon V-V de la figure 1,

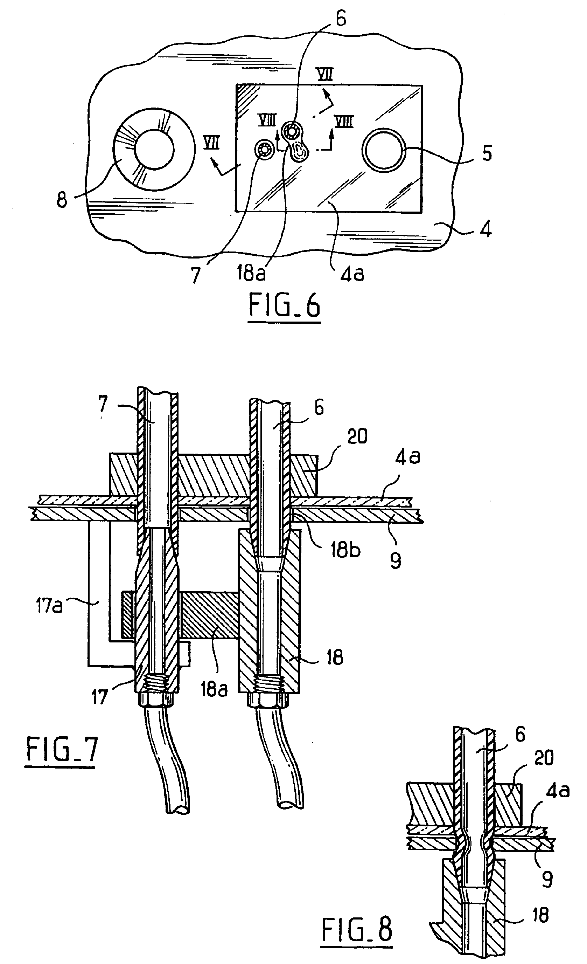

- la figure 6 est une vue rapprochée de dessus d'une partie du support,
- la figure 7 est une vue en coupe selon VII-VII de la figure 6,
- la figure 8 est une vue en coupe selon VIII-VIII de la figure 6,

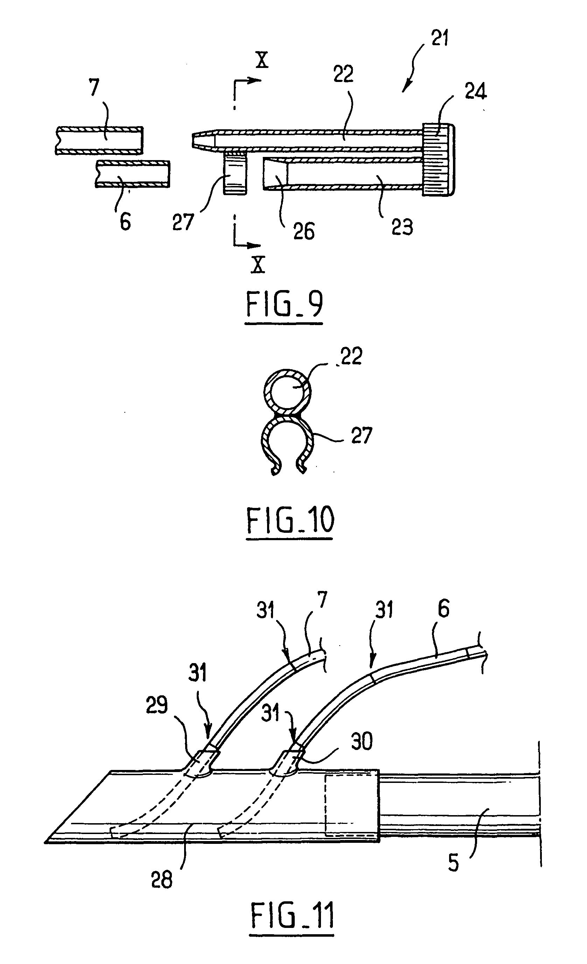

- la figure 9 est une vue en coupe longitudinale d'une prise rapide universelle,
- la figure 10 est une vue en coupe selon X-X de la figure 9,
- la figure 11 est une vue de côté d'un embout,

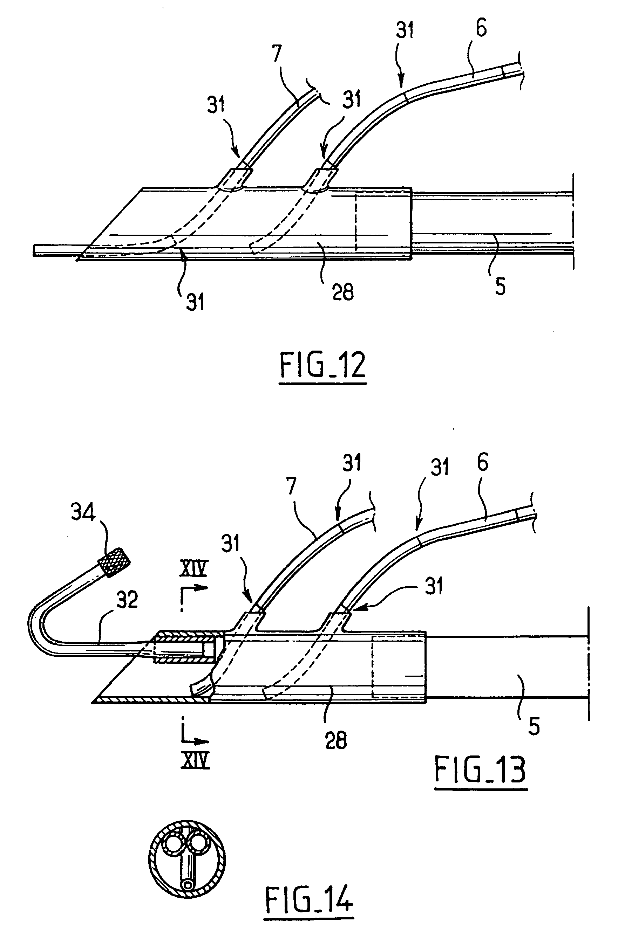

- la figure 12 est une vue analogue à la figure 11 après coulissement d'un tuyau flexible dans l'embout,
- la figure 13 est une vue analogue à la figure 11 après mise en place d'un tube coudé sur l'embout,
- la figure 14 est une vue en coupe selon XIV-XIV de la figure 13,

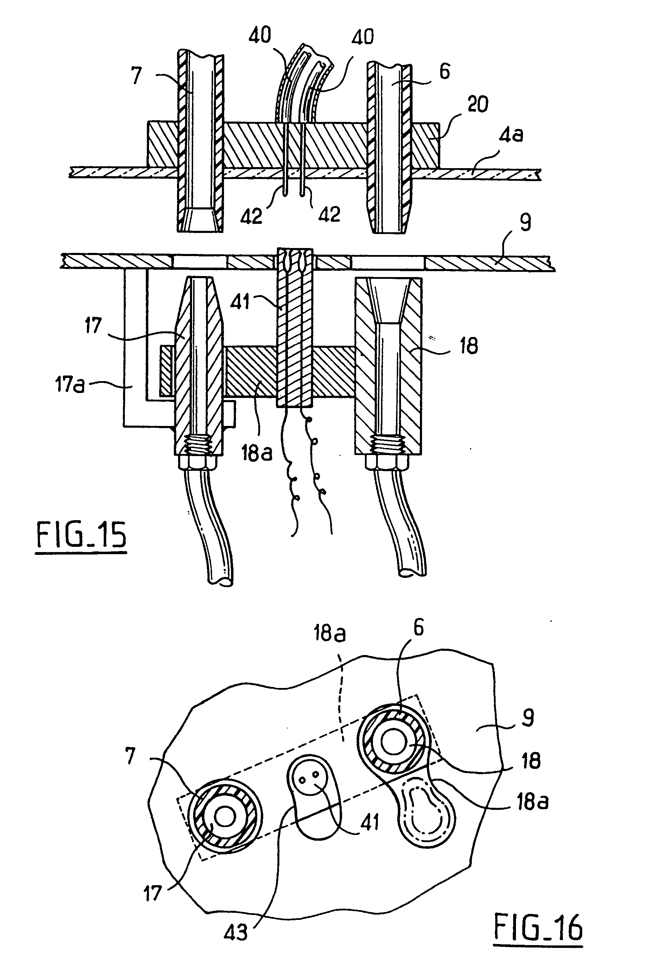

- la figure 15 est une vue analogue à la figure 7 d'un dispositif selon un autre mode de réalisation de l'invention, et
- la figure 16 est une vue analogue à la figure 6 correspondant au mode de réalisation de la figure 14.

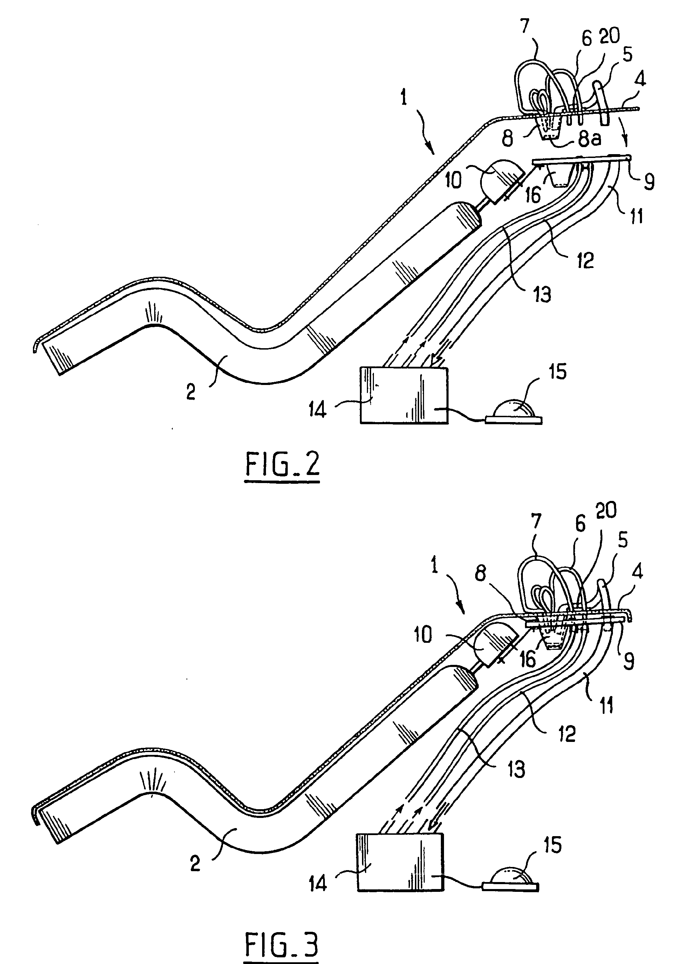

Sur la figure 1, on a représenté une feuille protectrice rectangulaire 1 de grandes dimensions qui est destinée, comme on le voit sur les figures 2 et 3, à recouvrir un fauteuil de cabinet dentaire 2.

Cette feuille protectrice 1 comprend un rabat 3 qui la recouvre sur ses deux tiers environ et qui est destiné à protéger un patient installé sur le fauteuil, en laissant dépasser la tête dudit patient.

Dans sa partie 4 non recouverte par le rabat, la feuille protectrice 1 est traversée par trois tuyaux flexibles 5, 6 et 7 dont l'un 5 présente un diamètre plus important.

Dans l'exemple représenté ici, les trois tuyaux flexibles 5,6 et 7 sont alignés et relativement rapprochés les uns des autres mais on préférera la version représentée à la figure 4bis dans laquelle le tuyau flexible de gros diamètre 5' est éloigné des deux tuyaux flexibles de petit diamètre 6' et 7', pour des raisons de protection contre les souillures lors du démontage de la feuille protectrice, comme cela a déjà été expliqué.

Conformément à une variante avantageuse, la feuille protectrice comporte, dans sa partie non recouverte par le rabat, une région centrale transparente 4a, destinée à faciliter la mise en place de la feuille, comme cela va être expliqué.

Les bases des deux tuyaux flexibles de petit diamètre 6 et 7 sont munies d'un raidisseur 20 qui peut être soit issu de moulage, soit rapporté sur la feuille protectrice, et qui permet de solidariser les deux tuyaux flexibles lors de la mise en place de la feuille, comme cela sera expliqué ultérieurement.

La figure 5 permet de mieux comprendre comment les trois tuyaux flexibles traversent la feuille protectrice dans sa région transparente 4a.

La paroi externe des tuyaux est raccordée de manière étanche à la feuille protectrice de telle sorte qu'il n'existe aucun autre passage d'une face à l'autre de la feuille protectrice que ceux constitués par les tuyaux eux-mêmes.

A côté des trois tuyaux flexibles 5, 6 et 7, la feuille protectrice 1 comporte une partie en creux 8 en forme de gobelet qui est réalisée d'un seul tenant avec ladite feuille et ne crée pas non plus le moindre passage d'une face à l'autre de la feuille protectrice.

Dans la version de la figure 4bis, la partie en creux 8' se trouve à distance des embouchures des petits tuyaux flexibles d'alimentation, afin d'éloigner toute source de contamination de ces tuyaux.

Les trois tuyaux flexibles 5, 6 et 7 font saillie de la face inférieure de la feuille protectrice sur une longueur d'environ 1 cm et, sur la face supérieure de la feuille, sur une longueur d'environ 1 m.

Comme on le voit sur les figures 2 et 3, le dispositif selon l'invention comprend également un support 9 qui est ici constitué par une tablette assujettie à la têtière 10 du fauteuil dentaire.

En dehors de la feuille protectrice, dont la partie supérieure et les éléments qui s'y rattachent sont destinés à être souillés lors d'une intervention, tous les autres éléments tels que le fauteuil, le support et ses équipements appartiennent à une zone qui n'est jamais contaminée lors des soins par le toucher ou par des projections de liquides biologiques.

La tablette 9 comprend trois conduits 11, 12, 13 de circulation de fluide, à savoir, un premier conduit 11 de gros diamètre correspondant au tuyau flexible de gros diamètre 5 de la feuille protectrice, et deux conduits 12, 13 de petit diamètre correspondant aux tuyaux flexibles 6, 7 de petit diamètre de la feuille protectrice.

Ces trois conduits 11, 12 et 13 sont reliés à un distributeur 14 qui est lui-même relié à une réserve d'eau, une réserve d'air comprimé et une pompe d'aspiration (non représentées).

Ainsi, le distributeur, qui est actionné par une pédale 15, envoie de l'eau dans le conduit 13 de petit diamètre et de l'air comprimé dans le conduit 12 de petit diamètre, et crée un vide dans le conduit 11 de gros diamètre.

Le support 9 comporte également un réceptacle 16 en forme de gobelet.

Comme on le voit sur les figures 2 à 4, lorsque l'on met en place la feuille protectrice 1 sur le fauteuil, la partie 4 de la feuille recouvre le support 9 avec sa région transparente 4a au-dessus des embouchures des conduits, ce qui permet de visualiser les tuyaux flexibles et les embouchures des conduits du support, facilitant ainsi leur raccordement. La partie en creux 8 de la feuille pénètre alors dans le réceptacle 16 du support et les parties saillantes des tuyaux flexibles se raccordent aux conduits du support.

Un aimant 16a plaque le fond métallisé 8a de la partie en creux 8 de la feuille contre le fond du réceptacle 16.

Le tuyau flexible d'aspiration 5 s'emmanche simplement dans le conduit d'aspiration 11 qui présente un diamètre intérieur sensiblement égal au diamètre extérieur du tuyau d'aspiration.

Les tuyaux flexibles d'alimentation en eau et en air comprimé se raccordent aux conduits du support comme représenté aux figures 7 et 8, grâce à des embouts 17 et 18.

L'embout 17 est fixe, supporté par une patte 17a assujettie au support.

L'embout 18 est mobile, monté sur un bras 18a pivotant autour de l'embout fixe 17, en regard d'une lumière 18b prévue dans le support et dont la forme est visible sur la figure 6.

Comme on le voit sur la figure 7, le tuyau flexible d'alimentation en eau 7 vient coiffer l'extrémité tronconique mâle de l'embout fixe 17 tandis que le tuyau flexible d'alimentation en air comprimé 6 s'engage dans l'extrémité tronconique femelle de l'embout mobile 18, qui est positionné comme représenté à la figure 6 en regard de la partie la plus large de la lumière 18b, grâce à un moyen de rappel (non représenté).

Dans un deuxième temps, on saisit le raidisseur 20 du côté supérieur de la feuille protectrice et on le fait pivoter autour du tuyau flexible d'alimentation en eau 7, dans la direction indiquée par la flèche sur la figure 6, ce qui déplace le tuyau flexible d'alimentation en air comprimé 6, lequel entraîne avec lui l'embout 18.

Le tuyau flexible d'air comprimé 6 arrive ainsi dans la partie étroite de la lumière 18b et se trouve pincé entre ses bords, comme représenté à la figure 8, ce qui assure sa rétention axiale.

Le sens de pivotement du bras 18a pour le verrouillage du tuyau flexible est choisi de préférence de telle manière que les tractions exercées sur ledit tuyau flexible par le praticien lorsqu'il travaille ne provoquent pas le retour dans la position de la figure 6 mais au contraire repoussent ledit tuyau entre les bords pinçants de la lumière.

Il faut noter que la mise en place de la feuille protectrice et des tuyaux flexibles, et en particulier le verrouillage du tuyau flexible d'alimentation en air comprimé, s'effectuent sans qu'aucun contact avec les conduits ne soit nécessaire, de sorte que la zone stérile ne risque à aucun moment d'être souillée.

De même, le démontage des trois tuyaux flexibles s'effectue par une simple traction exercée sur ceux-ci dans leur partie située du côté supérieur de la feuille protectrice 1, de sorte que le praticien peut déconnecter les conduits des tuyaux sans toucher ni le réceptacle ni les conduits, c'est-à-dire sans les contaminer.

Sur la figure 9, on a représenté une prise universelle 21 destinée à prendre place aux extrémités des deux tuyaux flexibles 6 et 7 d'alimentation en eau et en air comprimé.

Cette prise universelle comprend deux tubes rigides 22 et 23 de longueurs différentes qui aboutissent, à l'une de leurs extrémités, à un manchon de raccordement 24, par exemple à vis, prévu pour recevoir différents instruments.

A leur autre extrémité, les tubes rigides comprennent une partie conique mâle 25 et femelle 26, destinées à faciliter l'emmanchement du tuyau flexible d'eau 7 pour la première et l'introduction du tuyau flexible d'air comprimé 6 pour la seconde.

Le tuyau flexible d'eau 7 s'emmanche ainsi sur le tube le plus long 22 sur lequel il est retenu par simple friction du fait de l'élasticité dudit tuyau, la section extérieure du tube 22 étant très légèrement supérieure au diamètre intérieur du tuyau flexible 7.

Le tuyau flexible d'air comprimé 6 s'engage dans le tube le plus court 23 et se comprime radialement du fait que son diamètre extérieur est très légèrement supérieur au diamètre intérieur du tube 23. Le tuyau 6 est ensuite pincé par une patte 27 assujettie au tube d'eau 22 et située dans le prolongement du tube d'air 23.

Cette patte 27 assure la retenue du tuyau flexible d'amenée d'air comprimé.

Sur les figures 10 à 12, on a représenté un embout 28 qui permet l'utilisation simultanée des trois tuyaux flexibles lorsqu'il s'agit de les amener dans la bouche du patient.

Cet embout 28 est constitué par un tube cylindrique qui comporte latéralement deux cheminées obliques 29, 30 destinées à recevoir chacune un des tuyaux flexibles de petit diamètre 6,7.

Dans une variante non représentée, les cheminées sont parallèles à l'axe de l'embout et débouchent à l'arrière de ce dernier.

L'extrémité arrière de l'embout, c'est-à-dire celle située en amont des cheminées obliques, est destinée à recevoir le tuyau flexible d'aspiration 5 dont le diamètre extérieur correspond sensiblement au diamètre intérieur de l'embout.

Le lien entre le tuyau 5 et l'embout 28 peut être assuré par friction ou par tout autre moyen mais il est préférable que l'embout puisse pivoter autour de son axe par rapport au tuyau 5 pour des raisons d'ergonomie.

Les deux petits tuyaux flexibles demeurent en position dans l'embout par friction du fait de la correspondance de diamètre entre lesdits tuyaux flexibles et les parties de l'embout dans lesquelles ils sont engagés.

L'embout 28 constitue une pièce rigide que le praticien peut saisir facilement pour manoeuvrer simultanément les trois tuyaux flexibles.

Dans la position desdits tuyaux représentée à la figure 10, l'embout 28 sert principalement à l'aspiration du fait que les deux tuyaux flexibles 6 et 7 d'alimentation en eau et en air comprimé se situent en retrait de l'extrémité avant de l'embout.

Lorsqu'il désire amener de l'eau ou de l'air comprimé dans la bouche du patient, le praticien pousse le tuyau flexible de petit diamètre correspondant dans la cheminée et l'enfonce dans l'embout jusqu'à ce qu'il dépasse à l'extrémité avant de l'embout, comme on le voit à la figure 11 pour le tuyau flexible 7 d'alimentation en eau.

A cet effet, chaque tuyau flexible 6,7 comporte des marques annulaires 31 qui indiquent au praticien les deux positions, rentrée et sortie, que doit prendre ledit tuyau flexible dans l'embout, la marque considérée devant se trouver à la limite de l'embouchure de la cheminée correspondante.

Le praticien enfonce ensuite la pédale de commande 15 du distributeur 14 pour provoquer l'alimentation en fluide du tuyau flexible considéré ou des deux, si la pédale ne permet pas de distinguer les deux alimentations. La pompe d'aspiration fonctionne en permanence et est commandée séparément par un interrupteur au pied.

Avec une aspiration simultanée par le tuyau flexible de gros diamètre, le fluide demandé est délivré par le tuyau flexible dépassant de l'embout et l'autre fluide, s'il est envoyé dans le deuxième tuyau flexible, est immédiatement absorbé par le tuyau d'aspiration 5.

L'embout peut également être utilisé pour aspirer la salive dans la bouche du patient grâce à un tube amovible coudé flexible 32 qui est engagé dans un logement cylindrique 33 prévu à cet effet dans l'embout comme on le voit à la figure 13. Ce tube coudé est muni d'une crépine 34 à son extrémité libre, son autre extrémité débouchant dans le conduit d'aspiration.

Comme cela a déjà été expliqué, l'embout suspendu à la bouche du patient par le tube coudé 32 sert également de support pour les trois tuyaux flexibles qui demeurent ainsi à la disposition du praticien à proximité de la bouche du patient.

Comme on le voit à la figure 14, l'embout 28 peut comporter deux logements cylindriques 33, ce qui permet d'installer deux tubes coudés flexibles 32 lorsqu'on désire assécher plus efficacement la bouche du patient, à partir de deux points d'aspiration différents.

Sur les figures 15 et 16, on a représenté un dispositif sensiblement identique à celui de la figure 7, comportant en outre une ligne électrique constituée par des fils électriquement conducteurs 40 sur la feuille protectrice et par une prise électrique 41 sur le support.

Les fils 40 traversent la feuille protectrice dans sa région transparente 4a entre les deux tuyaux flexibles 6 et 7, en traversant également le raidisseur 20.

Ils sont terminés par des broches 42 aptes à pénétrer dans les orifices correspondants de la prise électrique 41.

Pour permettre le branchement des broches lorsque le bras mobile 18a se trouve en position de branchement, la prise électrique 41 est elle aussi assujettie au bras mobile et suit les mouvements des broches lors du verrouillage du tuyau flexible d'air comprimé.

Une lumière 43 est prévue à côté de la lumière 18a pour autoriser la rotation des broches.

Le raccordement électrique des fils 40 s'effectue donc comme celui des tuyaux flexibles, sans aucun contact direct avec le support qui demeure ainsi stérile.

Afin d'assurer le bon positionnement de la prise électrique 41 et de l'embout mobile 18 lors de la mise en place de la feuille protectrice, on prévoit un ressort de rappel et une butée (non représentés) sur le support.

Un avantage important du dispositif selon l'invention est qu'il comporte en lui-même tout l'équipement permettant au praticien de prodiguer ses soins, de sorte qu'il ne nécessite aucune structure autre que le siège et le support, ce qui facilite l'entretien de la zone stérile. En d'autres termes, le dispositif selon l'invention constitue un unit stérile jetable.

## Revendications

1. Dispositif pour intervention chirurgicale, destiné à assurer une protection des patients contre les contaminations, notamment en chirurgie dentaire, comportant une feuille protectrice imperméable (1) comprenant deux faces agencées pour délimiter respectivement une zone stérile et un milieu contaminé dans lequel peut se trouver un patient sur lequel une intervention doit être pratiquée, **caractérisé par le fait qu**'il comporte au moins un tuyau flexible (5, 6, 7) traversant la feuille de manière à constituer un passage entre la zone stérile et le milieu contaminé, la paroi extérieure dudit tuyau flexible étant réunie de façon étanche à la feuille protectrice.

2. Dispositif selon la revendication 1, **caractérisé par le fait qu'**il comporte, formée dans la feuille protectrice, au moins une partie en creux (8) pénétrant, lorsque la feuille protectrice est mise en place sur le support, dans un réceptacle du support apte à recevoir des instruments opératoires.

3. Dispositif selon l'une quelconque des revendications 1 et 2, **caractérisé par le fait que** la feuille protectrice comporte un opercule détachable au-dessus de sa partie en creux, constituant un témoin d'inviolabilité.

4. Dispositif selon l'une quelconque des revendications 1 à 3, **caractérisé par le fait que** la feuille protectrice (1) et le tuyau flexible (5,6,7) constituent un ensemble d'un seul tenant réalisé en un matériau peu coûteux de sorte que cet ensemble peut être jeté après chaque utilisation.

5. Dispositif selon l'une quelconque des revendications 1 à 4, **caractérisé par le fait qu'**il comporte deux tuyaux flexibles (6,7) sur la feuille protectrice, à savoir un tuyau flexible pour une alimentation en eau, un tuyau flexible pour une alimentation en air comprimé, destinés à se raccorder à des conduits d'alimentation (12, 13) sur le support.

6. Dispositif selon la revendication 5, **caractérisé par le fait qu'**il comporte, à la base des deux tuyaux flexibles, un raidisseur (20) qui réunit lesdites bases et les solidarise entre elles.

7. Dispositif selon l'une quelconque des revendications 1 à 6, **caractérisé par le fait qu'**il comporte un tuyau flexible d'aspiration (5) sur la feuille protectrice, destiné à se raccorder à un conduit d'aspiration (11) sur le support.

8. Dispositif selon les revendications 5 et 7, **caractérisé par le fait que** les embouchures des tuyaux flexibles d'alimentation et celle du tuyau flexible d'aspiration sont séparées par une distance importante ou par une cloison de séparation de manière à éviter que des gouttelettes de liquide contaminé provenant du tuyau flexible d'aspiration ne viennent souiller les embouchures des conduits d'alimentation au moment du retrait de la feuille protectrice.

9. Dispositif selon l'une quelconque des revendications 1 à 8, **caractérisé par le fait qu'**il comporte une ligne électrique constituée par un ou plusieurs fils conducteurs (40) sur la feuille protectrice, traversant cette dernière de façon étanche et munie de broches de contact (42) aptes à coopérer avec une prise électrique (41) du support lorsque la feuille protectrice est mise en place.

10. Dispositif selon l'une quelconque des revendications 1 à 9, **caractérisé par le fait qu'**il comporte au moins une gaine de protection constituée par un tuyau flexible de très gros diamètre raccordé de façon étanche par une de ses extrémités à la face de la feuille protectrice opposée au support et débouchant sur l'autre face de ladite feuille, pour permettre au praticien de manipuler des appareils non stérilisables, en les engageant dans la gaine.

11. Dispositif selon l'une quelconque des revendications 1 à 10, **caractérisé par le fait que** le support (9) est relié à au moins une pédale de commande (15) de la circulation du ou des fluides dans les conduits (11,12,13) dudit support.

12. Dispositif selon l'une quelconque des revendications 2 à 11, **caractérisé par le fait que** la partie en creux (8) de la feuille protectrice comporte un fond métallisé (8a), tandis que le réceptacle (16) du support (9) comporte un fond aimanté (16a).

13. Dispositif selon la revendication 5, **caractérisé par le fait que** le tuyau flexible d'alimentation en eau (7) se raccorde à un embout fixe (17) monté à l'extrémité du conduit d'alimentation en eau (13) et que le tuyau flexible d'alimentation en air comprimé (6) se raccorde à un embout mobile (18) monté à l'extrémité du conduit d'alimentation en air comprimé (12) et supporté par un bras (18a) pivotant autour de l'embout fixe (17), le support (9) étant muni d'une lumière (18b) en regard de laquelle l'embout mobile (18) se déplace lors du pivotement du bras, ladite lumière comportant une partie large permettant l'introduction du tuyau flexible d'alimentation en air comprimé et une partie étroite dont les bords pincent ledit tuyau flexible.

14. Dispositif selon les revendications 9 et 13, **caractérisé par le fait que** la prise électrique (41) du support est solidaire du bras pivotant (18a) et que le support comporte une seconde lumière (43) qui permet à ladite prise électrique de pivoter avec l'embout mobile (18) tout en restant accessible depuis le dessus du support pour le branchement des broches de contact.

15. Dispositif selon l'une quelconque des revendications 13 et 14, **caractérisé par le fait que** le bras pivotant (18a) est équipé d'un moyen de rappel, par exemple un ressort, qui assure son retour en position de branchement, c'est-à-dire avec son embout mobile en regard de la partie large de la lumière pour l'introduction du tuyau flexible d'air comprimé, une butée garantissant alors le bon positionnement du bras pivotant rappelé en position de branchement.

16. Dispositif selon l'une quelconque des revendications 1 à 15, **caractérisé par le fait qu'**il comporte, à l'extrémité du tuyau flexible (6,7), une prise universelle (21) destinée à recevoir tout instrument nécessitant un fluide pour son fonctionnement, le tuyau flexible (6,7) se raccordant à la prise universelle par simple emmanchement d'un tronçon d'extrémité du tuyau flexible dans un tube (22,23) de la prise, et éventuellement pincement de la paroi extérieure dudit tuyau flexible.

17. Dispositif selon la revendication 16, **caractérisé par le fait que** la prise universelle est en outre raccordée aux fils conducteurs et comporte des moyens de branchement conventionnels pour le montage d'un instrument nécessitant notamment une alimentation électrique.

18. Dispositif selon l'une quelconque des revendications 1 à 17, **caractérisé par le fait qu'**il comporte un embout dentaire (28) constitué par un cylindre muni de deux orifices latéraux (29,30), l'une des extrémités du cylindre étant destinée à recevoir l'extrémité d'un tuyau flexible d'aspiration (5), l'un des deux orifices latéraux (29) étant destiné à recevoir l'extrémité d'un tuyau flexible d'alimentation en eau (7), et l'autre orifice latéral (30) étant destiné à recevoir l'extrémité d'un tuyau flexible d'alimentation en air comprimé (6).

19. Dispositif selon la revendication 18, **caractérisé par le fait que** chaque tuyau flexible (6,7) est apte à coulisser dans l'orifice latéral (29,30 dans lequel il est engagé, de manière à pouvoir s'enfoncer dans l'embout pour faire saillie à l'extrémité de l'embout (28) opposée à celle recevant le tuyau d'aspiration (5).

20. Dispositif selon l'une quelconque des revendications 18 et 19, **caractérisé par le fait que** l'embout (28) comporte au moins un et de préférence deux tubes coudés flexibles (32) emmanchés dans l'embout à l'opposé du tuyau flexible d'aspiration (5), chaque tube coudé flexible étant muni d'une crépine (34) à son extrémité libre et étant destiné à être mis en place dans la bouche d'un patient pour aspirer sa salive.

## Claims

1. A surgical procedure device for protecting patients against contamination, in particular for dental surgery, the device comprising an impermeable protective sheet (1) having two faces arranged respectively to define a sterile zone and a contaminated medium in which there may be a patient on whom a procedure is to be performed, the device being **characterized by** the fact that it includes at least one flexible hose (5, 6, 7) passing through the sheet in such a manner as to constitute a passage between the sterile zone and the contaminated medium, the outer wall of said flexible hose being united in sealed manner with the protective sheet.

2. A device according to claim 1, **characterized by** the fact that it includes, formed in the protective sheet, at least one recessed portion (8) that penetrates, when the protective sheet is in position on the support, into a receptacle of the support suitable for receiving surgical instruments.

3. A device according to claim 1 or 2, **characterized by** the fact that the protective sheet includes a detachable film over its recessed portion, constituting a tamperproofing indicator.

4. A device according to any one of claims 1 to 3, **characterized by** the fact that the protective sheet (1) and the flexible hose (5, 6, 7) constitute a one-piece assembly made of an inexpensive material such that each assembly can be discarded after use.

5. A device according to any one of claims 1 to 4, **characterized by** the fact that it has two flexible hoses (6, 7) on the protective sheet, specifically one hose for feeding water and one hose for feeding compressed air, the hoses being for connection to feed ducts (12, 13) on the support.

6. A device according to claim 5, **characterized by** the fact that it includes a stiffener (20) at the bases of the two flexible hoses, which stiffener unites said bases and secures them to each other.

7. A device according to any one of claims 1 to 6, **characterized by** the fact that it includes, on the protective sheet, a suction hose (5) for connection to a suction duct (11) on the support.

8. A device according to claims 5 and 7, **characterized by** the fact that the openings of the feed hoses and the opening of the suction hose are separated by a large distance or by a separation partition so as to prevent drops of contaminated liquid coming from the suction hose dirtying the openings of the feed ducts when the protective sheet is removed.

9. A device according to any one of claims 1 to 8, **characterized by** the fact that it includes an electricity line constituted by one or more conductor wires (40) on the protective sheet, passing through the sheet in sealed manner and provided with contact pins (42) suitable for co-operating with an electricity outlet (41) on the support when the protective sheet is put into place.

10. A device according to any one of claims 1 to 9, **characterized by** the fact that it includes at least one protective sheath constituted by a flexible hose of very large diameter connected in sealed manner via one of its ends to the face of the protective sheet that is remote from the support and opening out into the other face of said sheet, to enable the practitioner to handle appliances that are not sterilizable by engaging them in the sheath.

11. A device according to any one of claims 1 to 10, **characterized by** the fact that the support (9) is connected to at least one control pedal (15) for controlling the flow of fluid(s) in the ducts (11, 12, 13) of said support.

12. A device according to any one of claims 2 to 11, **characterized by** the fact that the recessed portion (8) of the protective sheet includes a metal-covered bottom (8a) and the receptacle (16) in the support (9) includes magnetized bottom (16a).

13. A device according to claim 5, **characterized by** the fact that the water feed hose (7) is connected to a stationary endpiece (17) mounted at the end of the water feed duct (13) and that the compressed air feed hose (6) is connected to a moving endpiece (18) mounted at the end of the compressed air feed duct (12) and supported by an arm (18a) pivoting about the stationary endpiece (17), the support (9) being provided with a slot (18b) past which the moving endpiece (17) moves when the arm pivots, said slot including a wide portion enabling the compressed air feed hose to be inserted therein and a narrow portion with edges that clamp onto said hose.

14. A device according to claims 9 and 13, **characterized by** the fact that the electricity outlet (41) of the support is secured to the pivot arm (18a) and that the support includes a second slot (43) enabling said electricity outlet to pivot with the moving endpiece (18) while remaining accessible from above the support for connecting the contact pins.

15. A device according to claim 13 or claim 14, **characterized by** the fact that the pivot arm (18a) is fitted with return means, e.g. a spring, serving to urge it towards the connection position, i.e. with its moving endpiece in register with the large portion of the slot for inserting the compressed air hose, and abutments then guaranteeing that the pivot arm is properly positioned when returned to the connection position.

16. A device according to any one of claims 1 to 15, **characterized by** the fact that it includes a universal connector (21) at the end of the flexible hose (6, 7), the universal connector being suitable for receiving any instrument that requires fluid for its operation, the flexible hose (6, 7) being connected to the universal connector merely by engaging an end segment of the hose in a tube (22, 23) of the connector, and possibly clamping the outside wall of said flexible hose.

17. A device according to claim 16, **characterized by** the fact that the universal connector is also connected to the conductor wires and includes conventional connector means for mounting an instrument that requires an electricity power supply, in particular.

18. A device according to any one of claims 1 to 17, **characterized by** the fact that it includes a dental end fitting (28) constituted by a cylinder provided with two side orifices (29, 30), one of the ends of the cylinder being designed to receive the end of a suction hose (5), one of the two side orifices (29) being designed to receive the end of a water feed hose (7), and the other side orifice (30) being designed to receive the end of a compressed air feed hose (6).

19. A device according to claim 18, **characterized by** the fact that each hose (6, 7) is suitable for sliding in the side orifice (29, 30) in which it is engaged, so as to be capable of being pushed into the end fitting and project from the end of the fitting (28) remote from its end receiving the suction hose (5).

20. A device according to claim 18 or claim 19, **characterized by** the fact that the end fitting (28) includes at least one, and preferably two, angled flexible hoses (32) engaged in the end fitting at its end remote from the suction hose (5), each angled hose being provided with a strainer (34) at its free end and being designed for placing in the mouth of a patient to suck up saliva.

## Patentansprüche

1. Chirurgisches Gerät zum Schutz des Patienten gegen Kontamination, insbesondere bei zahnchirurgischen Eingriffen, welches eine undurchlässige Schutzfolie (1) aufweist, die zwei Seiten besitzt, welche so angeordnet sind, dass sie jeweils eine sterile Zone und eine kontaminierte Umgebung abgrenzen, in welcher sich ein Patient aufhalten kann, an welchem ein Eingriff vorzunehmen ist, **dadurch gekennzeichnet, dass** dieses mindestens einen flexiblen Schlauch (5, 6, 7) aufweist, welcher durch die Folie hindurch läuft, so dass er einen Durchgang zwischen der sterilen Zone und der kontaminierten Umgebung bildet, wobei die äußere Wand besagten flexiblen Schlauchs undurchlässig mit der Schutzfolie zusammengefügt ist.

2. Gerät nach Anspruch 1, **dadurch gekennzeichnet, dass** dieses mindestens eine in der Schutzfolie geformte Vertiefung (8) aufweist, welche, wenn die Schutzfolie auf der Auflage platziert wird, in ein Aufnahmeteil der Auflage dringt, welches zur Aufnahme von Operationsinstrumenten geeignet ist.

3. Gerät nach Anspruch 1 bis 2, **dadurch gekennzeichnet, dass** die Schutzfolie über ihrer Vertiefung einen lösbaren Deckel aufweist, welcher Unversehrtheit bezeugt.

4. Gerät nach einem der Ansprüche 1 bis 3, **dadurch gekennzeichnet, dass** die Schutzfolie (1) und der flexible Schlauch (5, 6, 7) ein Ganzes aus einem Stück bilden, welches aus einem kostengünstigen Material hergestellt ist, so dass dieses Ganze nach jeder Verwendung weggeworfen werden kann.

5. Gerät nach einem der Ansprüche 1 bis 4, **dadurch gekennzeichnet, dass** dieses zwei flexible Schläuche (6, 7) auf der Schutzfolie aufweist, und zwar einen flexiblen Schlauch für eine Versorgung mit Wasser, einen flexiblen Schlauch für eine Versorgung mit Druckluft, welche für den Anschluss an Versorgungsleitungen (12, 13) auf der Auflage bestimmt sind.

6. Gerät nach Anspruch 5, **dadurch gekennzeichnet, dass** dieses an dem unteren Abschnitt der beiden flexiblen Schläuche einen Versteifer (20) aufweist, welcher besagte untere Abschnitte verbindet und sie untereinander zusammenhält.

7. Gerät nach einem der Ansprüche 1 bis 6, **dadurch gekennzeichnet, dass** dieses einen flexiblen Saugschlauch (5) auf der Schutzfolie aufweist, welcher für den Anschluss an eine Saugleitung (11) auf der Auflage bestimmt ist.

8. Gerät nach Anspruch 5 und 7, **dadurch gekennzeichnet, dass** die Ausgangsöffnungen der flexiblen Versorgungsschläuche und jene des flexiblen Saugschlauchs durch eine erhebliche Entfernung oder durch eine Trennwand getrennt sind, um zu verhindern, dass aus dem flexiblen Saugschlauch stammende Tröpfchen kontaminierter Flüssigkeit die Ausgangsöffnungen der Versorgungsleitungen zum Zeitpunkt des Entfernens der Schutzfolie verunreinigen.

9. Gerät nach einem der Ansprüche 1 bis 8, **dadurch gekennzeichnet, dass** dieses eine elektrische Leitung aufweist, welche durch einen oder mehrere Leitungsdrähte (40) auf der Schutzfolie gebildet wird, durch Letztere in undurchlässiger Weise hindurchläuft und mit Kontaktstiften (42) ausgestattet ist, welche mit einer Steckdose (41) der Auflage zusammenwirken können, wenn die Schutzfolie platziert ist.

10. Gerät nach einem der Ansprüche 1 bis 9, **dadurch gekennzeichnet, dass** dieses mindestens eine Schutzhülle aufweist, die durch einen flexiblen Schlauch mit sehr großem Durchmesser gebildet wird, der in undurchlässiger Weise durch eines seiner Enden mit jener Seite der Schutzfolie verbunden ist, welche der Auflage gegenüber liegt, und auf die andere Seite besagter Folie mündet, um dem Arzt zu ermöglichen, nicht sterilisierbare Geräte zu handhaben, indem er sie in die Hülle einführt.

11. Gerät nach einem der Ansprüche 1 bis 10, **dadurch gekennzeichnet, dass** die Auflage (9) mit mindestens einem Pedal (15) zur Steuerung der Zirkulation des Fluids oder der Fluide in den Leitungen (11, 12, 13) besagter Auflage verbunden ist.

12. Gerät nach einem der Ansprüche 2 bis 11, **dadurch gekennzeichnet, dass** die Vertiefung (8) der Schutzfolie einen metallisierten Boden (8a) aufweist, wohingegen das Aufnahmeteil (16) der Auflage (9) einen magnetischen Boden (16a) enthält.

13. Gerät nach Anspruch 5, **dadurch gekennzeichnet, dass** der flexible Schlauch (7) zur Versorgung mit Wasser mit einem unbeweglichen Endstück (17) verbunden ist, welches an dem Ende der Leitung (13) zur Versorgung mit Wasser angebracht ist, und dass der flexible Schlauch (6) zur Versorgung mit Druckluft mit einem beweglichen Endstück (18) verbunden ist, welches an dem Ende der Leitung (12) zur Versorgung mit Druckluft angebracht ist und von einem Arm (18a) getragen wird, welcher sich um das unbewegliche Endstück (17) dreht, wobei die Auflage (9) mit einer Öffnung (18b) ausgestattet ist, der gegenüber sich das bewegliche Endstück (18) bei der Drehung des Arms bewegt, wobei besagte Öffnung einen weiten Teil umfasst, welcher das Einführen des flexiblen Schlauchs zur Versorgung mit Druckluft gestattet, und einen engen Teil, dessen Ränder besagten flexiblen Schlauch klemmen.

14. Gerät nach Anspruch 9 und 13, **dadurch gekennzeichnet, dass** die Steckdose (41) der Auflage mit dem Dreharm (18a) verbunden ist und dass die Auflage eine zweite Öffnung (43) besitzt, welche der Steckdose ermöglicht, sich mit dem beweglichen Endstück (18) zu drehen und gleichzeitig von der oberen Seite der Auflage zwecks Anschluss der Kontaktstifte zugänglich zu bleiben.

15. Gerät nach Anspruch 13 oder 14, **dadurch gekennzeichnet, dass** der Dreharm (18a) mit einem Rückführmittel, zum Beispiel mit einer Feder, ausgestattet ist, welches seine Rückkehr in eine Position zum Anschließen gewährleistet, das heißt, mit seinem beweglichen Endstück gegenüber dem weiten Teil der Öffnung zur Einführung des flexiblen Druckluftschlauchs, wobei ein Anschlag dann die gute Positionierung des in eine Position zum Anschließen rückgeführten Dreharms garantiert.

16. Gerät nach einem der Ansprüche 1 bis 15, **dadurch gekennzeichnet, dass** dieses, an dem Ende des flexiblen Schlauchs (6, 7), einen universellen Anschluss (21) aufweist, welcher dazu bestimmt ist, jedes Gerät aufzunehmen, welches ein Fluid für seine Funktion benötigt, wobei der flexible Schlauch (6, 7) mit dem universellen Anschluss durch simples Einpassen eines Endabschnitts des flexiblen Schlauchs in ein Rohr (22, 23) des Anschlusses, und eventuell durch Klemmen der äußeren Wand besagten flexiblen Schlauchs, verbunden wird.

17. Gerät nach Anspruch 16, **dadurch gekennzeichnet, dass** der universelle Anschluss weiterhin mit den Leitungsdrähten verbunden ist und herkömmliche Anschlussmittel für das Anbringen eines Geräts aufweist, welches insbesondere eine elektrische Versorgung benötigt.

18. Gerät nach einem der Ansprüche 1 bis 17, **dadurch gekennzeichnet, dass** dieses ein Zahnendstück (28) aufweist, welches von einem mit zwei seitlichen Öffnungen (29, 30) versehenen Zylinder gebildet wird, wobei eines der Enden des Zylinders dazu bestimmt ist, das Ende eines flexiblen Saugschlauchs (5) aufzunehmen, wobei eine der beiden seitlichen Öffnungen (29) dazu bestimmt ist, das Ende eines flexiblen Schlauchs (7) zur Versorgung mit Wasser aufzunehmen, und die andere seitliche Öffnung (30) dazu bestimmt ist, das Ende eines flexiblen Schlauchs (6) zur Versorgung mit Druckluft aufzunehmen.

19. Gerät nach Anspruch 18, **dadurch gekennzeichnet, dass** jeder flexible Schlauch (6, 7) in der Lage ist, in die seitliche Öffnung (29, 30) zu gleiten, in welche er eingeführt wird, damit er weit in dem Endstück vordringen kann, um an dem Ende des Endstücks (28) hervorzustehen, welches jenem gegenüberliegt, das den Saugschlauch (5) aufnimmt.

20. Gerät nach Anspruch 18 oder 19, **dadurch gekennzeichnet, dass** das Endstück (28) mindestens ein und vorzugsweise zwei flexible Knierohre (32) aufweist, welche in das Endstück gegenüber dem flexiblen Saugschlauch (5) eingepasst sind, wobei jedes flexible Knierohr mit einem Saugkopf (34) an seinem freien Ende versehen ist und dazu bestimmt ist, in dem Mund eines Patienten platziert zu werden, um dessen Speichel aufzusaugen.
